# FASCICULE DE BREVET EUROPEEN

(11) **EP 3 193 883 B1**
(45) Date de publication et mention de la délivrance du brevet: **23.02.2022**
(21) Numéro de dépôt: 15767132.2
(22) Date de dépôt: 18.09.2015
(51) Int. Cl.: A61K 31/65, A61K 31/7048, A61P 17/00

(54) **COMPOSITIONS COMPRENANT UN COMPOSE DE LA FAMILLE DES AVERMECTINES ET DE LA DOXYCYCLINE POUR LE TRAITEMENT DE LA ROSACEE**
ZUSAMMENSETZUNG MIT EINER VERBINDUNG AUS DER FAMILIE DER AVERMECTINEN UND DOXYCYCLIN ZUR BEHANDLUNG VON ROSACEA
COMPOSITION COMPRISING A COMPOUND FROM THE FAMILY OF AVERMECTINS AND DOXYCYCLINE FOR THE TREATMENT OF ROSACEA

(30) Priorité: 18.09.2014 FR 1458799
(43) Date de publication de la demande: 26.07.2017
(73) Titulaire: Galderma S.A., 6330 Cham (CH)
(72) Inventeur: JACOVELLA, Jean, F-06600 Antibes (FR)
(74) Mandataire: Nederlandsch Octrooibureau
(86) Numéro de dépôt international: PCT/EP2015/071402
(87) Numéro de publication internationale: WO 2016/042117

(56) Documents cités:
- EP-A1- 2 368 557
- WO-A1-2004/093886
- WO-A1-2014/049295
- US-A- 5 952 372

## Description

La présente invention est relative à des compositions utilisées dans le traitement et/ou le retardement de l'apparition des symptômes de la rosacée.

### ARRIERE PLAN TECHNOLOGIQUE DE L'INVENTION

La rosacée est une dermatose inflammatoire commune chronique caractérisée selon les formes par la présence d'érythème, de télangiectasies, œdème, de papules, des pustules, de lésions oculaires et parfois de rhinophyma. Elle est le plus souvent bilatérale et elle affecte principalement la partie médiane du visage, le front, le nez, le menton at les joues.

La rosacée se développe généralement chez les adultes entre l'âge de 30 à 50 ans et est beaucoup plus commune chez les gens au teint clair. Elle touche plus particulièrement les femmes, bien que cette affection soit généralement plus sévère chez l'homme. La rosacée est chronique et persiste des années avec des périodes d'exacerbation et de rémission.

La pathogénèse de la rosacée est mal connue. De nombreux facteurs peuvent être impliqués comme, par exemple, les facteurs psychologiques, environnementaux (exposition au soleil, température, humidité), émotionnels (stress), alimentaires (alcool, épices), les troubles hormonaux, gastro-intestinaux et vasculaires voire également une infection par *Helicobacter pilori* et la présence caractéristique du parasite *Demodex folliculorum* ou *Demodex brevis* chez les patients atteints de rosacée.

La rosacée peut être classée de la manière suivante :
- Type I: Rosacée érythématotélangiectasique, se caractérisant principalement par un érythème centrofacial persistant et des rougissements épisodiques ou bouffées de chaleur (« flush »). Souvent, ce type se caractérise également par un œdème, des plaques rugueuses et l'apparition de vaisseaux sanguins dilatés (télangiectasies) de même que par des sensations de brûlure et de piqûre.
- Type II: Rosacée papulopustulaire, se caractérisant par un érythème centrofacial persistant ainsi que par l'apparition de papules ou pustules centrofaciales transitoires. Ces symptômes sont parfois accompagnés de sensations de brûlure et de piqûre. Ce type peut suivre le type I ou se combiner à ce dernier.
- Type III: Rosacée phymateuse marquée par l'épaississement de la peau et l'apparition de nodules irréguliers. Bien que le nez soit souvent la région la plus touchée devenant très gros et couvert de boursouflures (« rhinophyma »), d'autres localisations sont également observées : menton, front, joues et oreilles. Ce type peut suivre le type I et II ou se combiner à ceux-ci.
- Type IV: Rosacée oculaire caractérisée par des yeux rouges et irrités pouvant larmoyer et semblant être injectés de sang. Les symptômes peuvent comprendre la sensation d'avoir un corps étranger dans l'œil, un larmoiement excessif, une sensibilité à la lumière, une vision floue, une sensation de brûlure, de sécheresse ou de piqûre, un prurit et une alacrymie.

Classiquement, la rosacée peut être traitée oralement ou topiquement par des antibiotiques tels que les tétracyclines, l'érythromycine, la clindamycine, mais aussi par la vitamine A, l'acide salicylique, des agents antifongiques, des stéroïdes, le métronidazole ou par l'isotrétinoïne dans les formes sévères ou encore par l'acide azélaïque. EP2368557 A1 décrit l'utilisation de la doxycycine en quantité sub-antibiotique pour traiter la rosacée et WO 2014/049295 A1 décrit l'utilisation d'une composition comprenant de la doxycycline et du laropiprant. Cependant, l'utilisation de ces antibiotiques ne permet pas de traiter et/ou prévenir efficacement tous les symptômes associés à la rosacée et peuvent souvent causer des effets secondaires et engendrer des problèmes d'intolérance chez de nombreux patients.

La rosacée peut également être traitée topiquement en utilisant de l'ivermectine ciblant le parasite *Demodex folliculorum* ou *Demodex brevis* tel que proposé dans le brevet US 5,952,372. WO 2004/093886 A1 décrit une composition comprenant 1% en poids d'ivermectine. Cependant des effets de résistance de ce parasite vis-à-vis de l'ivermectine pourraient survenir, diminuant ainsi l'efficacité d'un tel traitement ou le rendant inefficace.

Ainsi, force est de constater qu'il existe un besoin de fournir un traitement plus efficace de la rosacée qui ne présente pas d'effets secondaires et de problèmes d'intolérance pour le patient. En particulier, il n'existe pas à ce jour de traitement de la rosacée pouvant se substituer à ceux devenus moins efficaces ou inefficaces en raison des phénomènes de résistance.

### RESUME DE L'INVENTION

Il est du mérite du Demandeur d'avoir découvert qu'une association de l'ivermectine, avec la doxycycline permet un traitement plus efficace de la rosacée avec moins d'effets secondaires, notamment lorsque se développe une résistance du *Demodex,* particulièrement une résistance du *Demodex* à l'ivermectine.

En particulier, une telle association permet de réduire sensiblement la durée du traitement et d'obtenir une réduction plus importante des symptômes de la rosacée. Par ailleurs, l'association de ces deux actifs permet d'obtenir un avantage certain en termes d'efficacité et de tolérance permettant ainsi soit d'augmenter l'effet thérapeutique pour des doses similaires, soit de conserver le même effet thérapeutique tout en diminuant les doses. Enfin, une telle association permet de garantir l'efficacité du traitement lors de l'apparition d'une résistance de *Demodex* aux avermectines ou de repousser ou ralentir l'apparition de cette résistance.

L'objet de la présente invention est de proposer un kit comprenant de l'ivermectine, et de la doxycycline ou un de ses sels pharmaceutiquement acceptable, pour son utilisation dans la réduction, l'inhibition ou le ralentissement de l'apparition des symptômes liés à la rosacée, en particulier l'érythème, les papules, les pustules, les télangiectasies.

De préférence, la rosacée est de sous-type I, II et IV, et encore plus préférentiellement de sous-type II.

Dans un autre mode de réalisation préféré, le kit de l'invention est utilisées dans le traitement et/ou le retardement de l'apparition des symptômes d'une rosacée associée à/présentant une résistance de *Demodex* aux avermectines, préférentiellement à l'ivermectine.

Particulièrement, les compositions du kit utilisées dans l'invention sont destinées à être administrées par voie topique ou par voie orale.

Selon un mode particulier, les compositions du kit de l'invention sont destinées à être administrées chez un patient à une dose thérapeutique efficace d'ivermectine comprise entre 100 µg et 5 g, entre 100 µg et 3 g, entre 100 µg et 1 g, de préférence entre 150 µg et 500 mg, et encore plus préférentiellement entre 200 µg et 200 mg par jour.

Selon un autre mode particulier, les compositions du kit de l'invention sont destinées à être administrées chez un patient à une dose thérapeutique efficace de doxycycline ou d'un de ses sels pharmaceutiquement acceptable comprise entre 100 µg et 1 g, de préférence entre 100 µg et 500 mg, encore plus préférentiellement entre 10 et 300 mg par jour, et d'une manière encore plus préférée à une dose thérapeutique efficace de 40, 100 ou 200 mg par jour.

Un autre objet de l'invention concerne un kit comprenant (a) une composition comprenant l'ivermectine et (b) une composition comprenant de la doxycycline ou un de ses sels pharmaceutiquement acceptable, comme produit de combinaison pour son utilisation simultanée, séparée ou étalée dans le temps dans le traitement et/ou le retardement de l'apparition des symptômes de la rosacée.

De préférence, la rosacée est de sous-type I, II et IV, et encore plus préférentiellement de sous-type II.

Dans un autre mode de réalisation préféré, les kits selon l'invention sont utilisés dans le traitement et/ou le retardement de l'apparition des symptômes d'une rosacée associée à/présentant une résistance de *Demodex* aux avermectines, préférentiellement à l'ivermectine.

Dans un mode de réalisation particulier, la composition du kit comprenant l'ivermectine, est destinée à être administrée à une dose thérapeutique efficace comprise entre 100 µg et 5 g, entre 100 µg et 3 g, entre 100 µg et 1 g, de préférence entre 150 µg et 500 mg, et encore plus préférentiellement entre 200 µg et 200 mg par jour, et la composition du kit comprenant de la doxycycline ou un de ses sels pharmaceutiquement acceptable est destinée à être administrée à une dose thérapeutique efficace comprise entre 100 µg et 1 g, de préférence entre 100 µg et 500 mg, encore plus préférentiellement entre 10 et 300 mg par jour, et d'une manière encore plus préférée à une dose thérapeutique efficace de 40, 100 ou 200 mg par jour.

### DESCRIPTION DETAILLEE DE L'INVENTION

Les inventeurs ont identifié une nouvelle utilisation d'un kit dans le traitement et/ou le retardement de l'apparition des symptômes de la rosacée et ont découvert de façon surprenante que l'association des deux actifs qui sont l'ivermectine, et la doxycycline permettaient de traiter et/ou retarder l'apparition des symptômes de la rosacée. En particulier, il a été démontré qu'une telle association était efficace pour traiter les différents types de rosacée y compris lorsque des phénomènes de résistance de *Demodex* étaient observés.

L'invention concerne donc un kit comprenant l'ivermectine, et de la doxycycline ou un de ses sels pharmaceutiquement acceptable, pour son utilisation dans le traitement et/ou le retardement de l'apparition des symptômes de la rosacée, préférentiellement la rosacée de sous-type I, II et IV tels que définis précédemment, et encore plus préférentiellement la rosacée de sous-type II.

### Actifs

La classe des avermectines est un groupe de lactones macrocycliques produit par la bactérie *Streptomyces avermitilis* (Reynolds JEF (Ed) (1993) Martindale. The extra pharmacopoeia. 29th Edition. Pharmaceutical Press, London). Parmi ces lactones macrocycliques appartenant à la classe des avermectines, on peut citer l'ivermectine, l'avermectine, l'abamectine, la doramectine, l'éprinomectine, la selamectine, l'aversectine B, AB ou C, l'émamectine Blb et leurs dérivés, ou la latidectine. Selon l'invention, le composé de la famille des avermectines est l'ivermectine.

L'ivermectine est un mélange de 22,23-dihydroavermectine B₁ₐ et 22,23-dihydroavermectine B_{1b}. L'ivermectine contient majoritairement du 22,23-dihydroavermectine Bia.

L'ivermectine est connue dans l'art antérieur pour ses propriétés antiparasitaires et anthelminthiques. Au milieu des années 80, la molécule a été présentée comme médicament antiparasitaire de large spectre à usage vétérinaire (CAMPBELL, W.C., et al., (1983). Ivermectin: a patent new antiparasitic agent. Science, 221, 823-828. ). Elle est efficace contre la plupart des vers intestinaux communs (excepté les ténias), la plupart des acarides, et quelques poux. Elle présente une affinité importante pour les canaux chlorure glutamate-dépendants, notamment ceux dépendant du neuromédiateur GABA (acide gamma-amino-butyrique), présents dans les cellules nerveuses et musculaires des invertébrés, lui conférant une activité antiparasitaire. Plus particulièrement, sa fixation sur ces canaux favorise une augmentation de la perméabilité membranaire aux ions chlorures entraînant une hyperpolarisation de la cellule nerveuse ou musculaire. Il en résulte une paralysie neuromusculaire pouvant entraîner la mort de certains parasites. L'ivermectine interagit également avec d'autres canaux chlore.

L'ivermectine est utilisée classiquement dans le traitement dermatologique des manifestations endoparasitaires telles que l'onchocercose et la myase. Les brevets US 6,133,310 et US 5,952,372 décrivent également l'utilisation de l'ivermectine dans le traitement de la rosacée afin de réduire et éliminer le parasite *Demodex folliculorum* présent sur la peau des patients. Cependant, des phénomènes de résistance sont possibles, rendant de tels traitements plus ou moins inefficaces dans le traitement de la rosacée associée à/présentant une résistance de *Demodex* à l'ivermectine.

La doxycycline est une molécule des cyclines connue de l'art antérieur pour ses propriétés antibiotiques. Elle désigne l'alpha -6-désoxy-5-oxytétracycline ou le 1,4,4a,5,5a,6,11,12a-octahydro-3,5,10,12,12a-pentahydroxy-6-methyl-1,11-dioxo-2-naphthacene-carboxamide de formule :

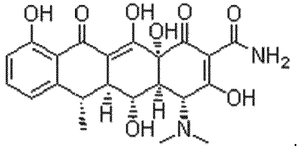

Dans le contexte de la présente invention, la doxycycline comprend également ses sels pharmaceutiquement acceptables. L'expression "sel(s) pharmaceutiquement acceptable(s)" désigne les sels d'un composé d'intérêt qui possèdent l'activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, ptoluènesulfonate et le pamoate (c'est-à-dire, 1,1'- méthylène-bis-(2-hydroxy-3-naphtoate)). Des sels de base adaptés comprennent, mais ne sont pas limités à, des sels d'aluminium, calcium, lithium, magnésium, potassium, sodium, zinc, et diéthanolamine.

Une liste de sels pharmaceutiquement acceptables est notamment publiée dans la revue de Berge et al. (J. Pharm. Sci. 1977, 66(1), 1-19).

Les différents sels pharmaceutiquement acceptables ou les différentes formes de la doxycycline utilisés dans la présente invention sont le chlorhydrate de doxycycline, le monohydrate de doxycycline et l'hyclate de doxycycline.

La présence de la doxycycline comme second actif dans les compositions du kit de l'invention permet avantageusement, par ses propriétés antibiotiques, de traiter une rosacée associée à/présentant une résistance de *Demodex* à un composé de la famille des avermectines, notamment l'ivermectine. En effet, une composition comprenant comme seul actif l'ivermectine est ou peut devenir inefficace pour traiter une rosacée lorsque le *Demodex* devient résistant à l'ivermectine. La combinaison avec la doxycycline est donc avantageuse pour retarder l'apparition de la résistance ou pour agir contre les *Demodex* résistants.

Un autre objet particulier de l'invention concerne la doxycycline ou un de ses sels pharmaceutiquement acceptable pour une utilisation en combinaison avec l'ivermectine dans le traitement et/ou le retardement de l'apparition des symptômes d'une rosacée associée à/présentant une résistance de *Demodex,* particulièrement une résistance de *Demodex* aux avermectines, préférentiellement à l'ivermectine.

Par l'expression «rosacée associée à/présentant une résistance de *Demodex* aux avermectines », on entend une rosacée présentant des *Demodex* résistants au traitement aux avermectines. Ainsi, un traitement à base d'un composé de la famille des avermectines uniquement est devenu moins efficace ou inefficace suite à cette résistance.

### Additifs

Les compositions du kit de l'invention peuvent comprendre un milieu physiologiquement acceptable, c'est-à-dire un milieu compatible avec la peau, les muqueuses et/ou les phanères. Les compositions du kit de l'invention peuvent comprendre également un véhicule pharmaceutiquement ou cosmétiquement acceptable, c'est-à-dire un véhicule adapté pour une utilisation en contact avec des cellules humaines, sans toxicité, intolérance, irritation, réponse allergique indue et similaire, et proportionné à un rapport avantage/risque raisonnable.

Les compositions du kit de l'invention peuvent comprendre en outre tout additif ou adjuvant usuellement utilisé dans le domaine pharmaceutique, dermatologique ou cosmétique, compatible avec l'ivermectine et la doxycycline en présence.

On peut citer notamment des séquestrants, des chélatants des antioxydants, des filtres solaires, des conservateurs, par exemple la DL-alpha-tocophérol, des charges, des électrolytes, des humectants, des colorants, de bases ou d'acides usuels, minéraux ou organiques, des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, des émulsionnants, des gélifiants, des agents épaississants, des agents tampons, des excipients lipophiles, des agents délitants, des agents solubles, des agents de compression ou un mélange de ceux-ci. Bien entendu, l'homme du métier veillera à choisir ce ou ces éventuels composés complémentaires, et/ou leur quantité, de manière telle que les propriétés avantageuses des compositions du kit selon l'invention ne soient pas, ou substantiellement pas, altérées.

Comme conservateurs, on peut citer à titre d'exemple, des ammoniums quaternaires tels que le chlorure de benzalkonium; le phénoxyéthanol; l'alcool benzylique; la diazolidinylurée; les parabens, tels que le méthylparabène, le propylparabène ou le butylparabène; l'acide benzoïque et ses sels sodiques ou potassique tels que le benzoate de sodium; l'acide sorbique et ses sels sodiques ou potassique tels que le sorbate de potassium; des dérivés mercuriels tels que les sels de phénylmercure (acétate, borate ou nitrate) ou le thiomersal; ou leurs mélanges.

Comme agents humectants, on peut citer en particulier, la glycérine et le sorbitol.

Comme agents chélatants, on peut citer à titre d'exemple, l'acide éthylènediaminetétracétique (EDTA), ainsi que ses dérivés ou ses sels, la dihydroxyethylglycine, l'acide citrique, l'acide tartrique ou leurs mélanges.

Comme agents propénétrants, on peut citer en particulier, le propylène glycol, le dipropylène glycol, le propylène glycol dipélargonate, le lauroglycol et l'ethoxydiglycol.

Comme matières grasses utilisables dans l'invention, on peut citer de manière non limitative les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclomethicone) et les huiles fluorées (perfluoropolyethers). On peut aussi utiliser comme matières grasses des alcools gras tels que l'alcool cétylique, des acides gras, des cires et des gommes en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40; les esters d'acide gras et de polyol tels que le stéarate de glycéryle, le tristéarate de sorbitane et les stéarates de sorbitane oxyéthylénés disponibles sous les dénominations commerciales Tween 20 ou Tween 60, par exemple; et leurs mélanges.

Comme gélifiants, à titre d'exemples non limitatifs, on peut citer la famille des polyacrylamides tels que le mélange Sodium acryloyldiméthyltaurate copolymer / isohexadecane / polysorbate 80 vendu sous le nom Simulgel^{™} 600 par la société Seppic^{™}, le mélange polyacrylamide / isoparaffine C13-14 / laureth-7 comme, par exemple, celui vendu sous le nom de Sepigel 305^{™} par la société Seppic^{™}, la famille des polymères acryliques couplés à des chaînes hydrophobes tel que le PEG-150 / decyl / SMDI copolymère vendu sous le nom de Aculyn 44^{™} (polycondensat comprenant au moins comme éléments, un poléthylèneglycol à 150 ou 180 moles d'oxyde d'éthylène, de l'alcool décylique et du méthylène bis(4-cyclohexylisocyanate) (SMDI), à 35 % en poids dans un mélange de propylèneglycol (39 %) et d'eau (26 %)), la famille des amidons modifiés tels que l'amidon de pomme de terre modifié vendu sous le nom de Structure Solanace^{™} ou bien leurs mélanges.

Les gélifiants préférés sont issus de la famille des polyacrylamides tel que le Simulgel 600^{™} ou le Sepigel 305^{™} ou leurs mélanges.

Les compositions du kit peuvent comprendre un ou plusieurs agents épaississants, permettant d'obtenir une viscosité adéquate, sélectionnés dans le groupe constitué des polysaccharides, des dérivés de cellulose et des polymères carboxyvinyliques de type Carbopol^{®}. Ces polymères comprennent, sans y être limités, le Carbopol^{®} 934P, Carbopol^{®} 71G, Carbopol^{®} 971P et Carbopol^{®} 974P.

Les compositions du kit peuvent comprendre un ou plusieurs agents épaississants sélectionnés parmi les dérivés de cellulose. Les dérivés de cellulose utilisables en tant qu'agents épaississants, comprennent, sans y être limités, la méthylcellulose, l'éthylcellulose, l'éthylméthylcellulose, la carboxyméthylcellulose, la carboxyméthylcellulose sodique et des hydroxyalkylcelluloses tels que l'hydroxyéthylcellulose, l'hydroxyméthylcellulose, l'hydroxypropylcellulose et l'hydroxypropylméthylcellulose, et une combinaison de ceux-ci.

Les compositions du kit peuvent comprendre un ou plusieurs agents épaississants sélectionnés parmi les polysaccharides. Les polysaccharides utilisables en tant qu'agent épaississant comprennent, sans y être limités, la gomme xanthane, la gomme adragante, les carraghénanes tels que les λ-carraghénane, le κ-carraghénane ou le ι-carraghénane, les galactomannanes tels que la farine de graines de caroube, la farine de graines de guar ou la farine de graines de tara, la gomme gellane, la gomme arabique, la gomme karaya, les pectines, l'amidon et ses dérivés obtenus par estérification ou éthérification, et le tamarin, et une combinaison de ceux-ci.

Les compositions du kit peuvent également comprendre, en tant qu'agents épaississants, un mélange d'un ou plusieurs dérivés de cellulose et d'un ou plusieurs polysaccharides.

Les compositions du kit selon l'invention peuvent en outre comprendre au moins un agent délitant tel que le carboxyméthylamidon sodique, la carboxyméthylcellulose de sodium réticulée ou la polyvinylpyrrolidone réticulée, et plus avantageusement la polyvinylpyrrolidone réticulée.

Les compositions du kit selon l'invention peuvent comprendre en outre au moins un agent soluble de type polyol (ou sucre-alcool). Un polyol (ou sucre-alcool) est une forme hydrogénée de sucre dans lequel le groupe carbonyle (i.e. aldéhyde ou cétone) a été réduit en un groupe hydroxyle primaire ou secondaire. En particulier, l'agent soluble diluant est choisi parmi le groupe comprenant le mannitol, le xylitol, le sorbitol, le maltitol et leur mélange. Ledit agent soluble est utilisé sous formes de granulés, de poudres ou un mélange de granulés et de poudres.

Les compositions du kit de l'invention peuvent comprendre des agents de compression pour la réalisation technique de comprimés tels que:
- agents lubrifiants tels que le stéarate de magnésium, l'acide stéarique, le monostéarate de glycérol, les polyoxyethylèneglycols ayant un poids moléculaire de 400 à 7 000 000, l'huile de ricin hydrogénée, le béhénate de glycérol, les glycérides mono-, bi-ou trisubstitués, le sodium stéaryl fumarate ;
- agents d'écoulement, tels que la silice colloidale ou toute autre silice, par exemple le produit commercialisé par Degussa sous la marque Aerosil ;
- liants tels que l'amidon, tampons, absorbants, diluants tels que lactose ainsi que tout autre additif pharmaceutiquement acceptable.

### Application

L'administration peut être effectuée par voie orale, topique, oculaire, intraoculaire, intraveineuse, parentérale, sous-cutanée, épicutanée, intra-dermique, transdermique, intramusculaire, entérale, rectale, intranasale, sublinguale, buccale, intra-respiratoire ou par inhalation nasale.

Lorsque l'administration est réalisée par voie topique sur la peau ou par voie oculaire sur l'œil, la composition peut être qualifiée de composition dermatologique.

Les collyres sont particulièrement adaptés à la voie oculaire. La composition administrable par voie topique est plus particulièrement destinée au traitement de la peau et des muqueuses. On entend par application topique, le fait d'appliquer ou d'étaler la composition du kit selon l'invention à la surface de la peau ou d'une muqueuse.

Parmi ces voies d'administration, la voie orale, la voie topique et la voie oculaire sont particulièrement préférées et la voie orale et la voie topique sont encore plus préférées.

Il est envisagé, pour une application topique, des compositions sous forme de solutions, de lotions, de gels, d'onguents, d'émulsions de consistance liquide ou semi-liquide du type lait, obtenues par dispersion d'une phase grasse dans une phase aqueuse (H/E) ou inversement (E/H), ou de poudres, de tampons imbibes, de sprays, de suspensions ou émulsions de consistance molle, semi-liquide ou solide du type crème, pommade ou encore de micro-émulsions, de micro-capsules, de micro-particules ou de dispersions vésiculaires de type ionique et/ou non ionique. Il peut également se présenter sous forme de microsphères ou nanosphères ou vésicules lipidiques ou polymériques ou de patches polymériques et d'hydrogels permettant une libération contrôlée. Ces compositions sont préparées selon les méthodes usuelles.

Pour une administration par voie topique, la composition se présente avantageusement sous la forme d'une pommade, d'une crème, d'une lotion ou d'un gel et une formulation particulièrement adaptée est celle proposée dans la demande WO 2004/093886.

Les compositions du kit de la présente invention peuvent aussi se présenter sous toutes les formes galéniques normalement utilisées pour une administration orale, notamment sous forme de comprimés, de gélules, de pilules, de poudre ou de toute forme pour préparation orale solide ou sous toute forme de préparation buvable. La composition pharmaceutique comprend généralement un milieu physiologiquement acceptable, par exemple pour la préparation de comprimés ou de gélules ou pour une préparation buvable telle que les véhicules utilisés de façon tout à fait classique.

Les compositions pharmaceutiques du kit selon la présente invention sont de taille acceptable pour l'administration orale classique. Ainsi, on préfère les compositions d'un poids inférieur à 800 mg, de préférence inférieur à 500 mg, et plus particulièrement inférieur à 400 mg (comme par exemple des compositions dont le poids est de 150 mg, 200 mg ou de 250 mg).

De manière avantageuse, pour une administration par voie orale, la composition comprend un comprimé, une gélule, une pilule, des granulés ou se présente sous forme de poudre.

Dans un mode de réalisation, le terme " traitement " ou " traiter " désigne une amélioration, la prophylaxie, ou l'inversion d'une maladie ou d'un trouble, ou au moins d'un symptôme pouvant être discerné de celui-ci. Dans un autre mode de réalisation, " traitement " ou " traiter " désigne une amélioration, la prophylaxie, ou l'inversion d'au moins un paramètre physique mesurable associé à la maladie ou au trouble étant traité, qui n'est pas nécessairement discernable chez ou par le sujet traité. Dans un autre mode de réalisation supplémentaire, " traitement " ou " traiter " désigne l'inhibition ou le ralentissement de la progression d'une maladie ou un trouble, physiquement, par exemple, la stabilisation d'un symptôme discernable, physiologiquement, par exemple, la stabilisation d'un paramètre physique, ou les deux. Dans un autre mode de réalisation, " traitement " ou " traiter " désigne le retard de l'apparition d'une maladie ou trouble.

Dans certains modes de réalisation, des composés d'intérêt sont administrés en tant que mesure préventive. Dans le présent contexte, cette mesure préventive désigne une réduction du risque d'acquisition d'une maladie ou un trouble spécifié mais aussi une réduction, une inhibition ou un ralentissement de l'apparition des symptômes liés à cette maladie qui est la rosacée. Des symptômes caractéristiques de la rosacée sont par exemple les érythèmes, les papules, les pustules et les télangiectasies.

Au sens de la présente invention, par « patient » on entend tout mammifère, et plus particulièrement les êtres humains, hommes ou femmes.

La quantité réellement administrée de l'ivermectine et de doxycycline ou d'un sel pharmaceutiquement acceptable de celle-ci et éventuellement d'autres additifs à mettre en œuvre selon l'invention dépend de l'effet thérapeutique ou cosmétique recherché, et peut donc varier dans une large mesure. L'homme de l'art, en particulier le médecin peut aisément, sur la base de ses connaissances générales déterminer les quantités appropriées. Ainsi, et selon une forme de réalisation préférée, la ou les composition(s) pharmaceutique(s) sont administrées 1 à 2 fois/jour. De préférence, le traitement peut avoir une durée allant de 1 semaine à 6 mois, renouvelable, et de préférence de 2 semaines à 4 mois.

Les cures peuvent être renouvelées en cycle avec ou sans période de repos.

Dans les compositions du kit selon l'invention, la dose thérapeutique efficace de l'ivermectine administrée est de 100 µg à 5 g, de 100 µg et 3 g, de 100 µg à 1 g, de préférence de 150 µg à 500 mg, et encore plus préférentiellement de 200 µg à 200 mg par jour.

Dans les compositions du kit selon l'invention, la dose thérapeutique efficace de doxycycline ou d'un de ses sels pharmaceutiquement acceptable administré est comprise entre 100 µg et 1 g, de préférence entre 100 µg et 500 mg, encore plus préférentiellement entre 10 et 300 mg par jour, et d'une manière encore plus préférée à une dose thérapeutique efficace de 40, 100 ou 200 mg par jour.

Dans le contexte de l'invention, on entend par « dose thérapeutique efficace » la dose thérapeutique qui empêche, supprime ou réduit les effets délétères de la rosacée traitée chez le patient. Il est entendu que la dose administrée peut être adapté par l'homme du métier en fonction du patient, du type de rosacée, du mode d'administration, etc

### Kits

L'invention concerne un kit comprenant (a) l'ivermectine, et (b) une composition comprenant de la doxycycline ou un de ses sels pharmaceutiquement acceptable, comme produit de combinaison pour une utilisation simultanée, séparée ou étalée dans le temps dans le traitement et/ou le retardement de l'apparition des symptômes de la rosacée.

De préférence, le kit selon l'invention est utilisée dans le traitement et/ou le retardement de l'apparition des symptômes de la rosacée de sous-type I, II et IV, avantageusement de sous-type II.

Particulièrement, le kit selon l'invention est utilisé dans le traitement et/ou le retardement de l'apparition des symptômes d'une rosacée associée à/présentant une résistance de *Demodex,* particulièrement une résistance de *Demodex* aux avermectines, préférentiellement à l'ivermectine.

Les compositions du kit peuvent être conditionnées séparément dans des récipients distincts pouvant se présenter sous diverses formes. Il peut s'agir notamment de tubes ou de flacons.

Selon un mode de réalisation, les deux récipients sont indépendants l'un de l'autre. Selon un mode de réalisation particulier, les deux compositions sont conditionnées dans un dispositif unitaire, lesdits récipients formant des compartiments solidaires l'un de l'autre.

Dans un mode de réalisation particulier, la composition du kit comprenant l'ivermectine est destinée à être administrée à une dose thérapeutique efficace comprise entre 100 µg et 5 g, entre 100 µg et 3 g, entre 100 µg et 1 g, de préférence entre 150 µg et 500 mg, et encore plus préférentiellement entre 200 µg et 200 mg par jour ; et la composition comprenant de la doxycycline ou un de ses sels pharmaceutiquement acceptable est destinée à être administrée à une dose thérapeutique efficace comprise entre 100 µg et 1 g, de préférence entre 100 µg et 500 mg, encore plus préférentiellement entre 10 et 300 mg par jour, et d'une manière encore plus préférée à une dose thérapeutique efficace de 40, 100 ou 200 mg par jour. L'ivermectine et la doxycycline ou un de sels pharmaceutiquement acceptable sont de préférence administrés par voie topique, orale ou oculaire.

Dans un premier mode de réalisation, l'ivermectine et la doxycycline ou un de sels pharmaceutiquement acceptable sont administrés par voie topique.

Dans un second mode de réalisation, l'ivermectine, et la doxycycline ou un de sels pharmaceutiquement acceptable sont administrés par voie orale.

Dans un troisième mode de réalisation, l'ivermectine est administrée par voie topique et la doxycycline ou un de sels pharmaceutiquement acceptable est administré par voie orale.

## Revendications

1. Kit comprenant (a) une composition comprenant l'ivermectine destinée à être administrée à une dose thérapeutique efficace comprise entre 100 µg et 5 g par jour, et (b) une composition comprenant de la doxycycline ou un de ses sels pharmaceutiquement acceptable, destinée à être administrée à une dose thérapeutique efficace de 40, 100, ou 200 mg par jour, comme produit de combinaison pour son utilisation simultanée, separée, ou étalée dans le temps dans le traitement et/ou le retardement de l'apparition des symptômes de la rosacée

2. Kit pour son utilisation selon la revendication 1, dans le traitement et/ou le retardement de l'apparition des symptômes de la rosacée de sous-type I, II et IV, de préférence de sous-type II

3. Kit pour son utilisation selon l'une quelconque des revendications 1 ou 2, dans le traitement et/ou le retardement de l'apparition des symptômes d'une rosacée associée à/présentant une résistance de *Demodex* aux avermectines, préférentiellement à l'ivermectine

4. Kit pour son utilisation selon l'une quelconque des revendications 1 à 3, **caractérisée en ce que** la composition comprenant de l'ivermectine est destinée à une administration par voie topique

5. Kit pour son utilisation selon l'une quelconque des revendications 1 à 4, **caractérisée en ce que** la composition comprenant de la doxycycline ou un de ses sels pharmaceutiquement acceptable est destinée à une administration par voie orale

6. Kit pour son utilisation selon l'une quelconque des revendications 1 à 5, **caractérisée en ce que** la composition du kit comprenant de la doxycycline ou un de ses sels pharmaceutiquement acceptable est destinée à être administrée a une dose thérapeutique efficace de 40 mg de la doxycycline par jour

## Patentansprüche

1. Kit, welches (a) eine Zusammensetzung umfasst, die Ivermectin umfasst, die zur Verabreichung einer therapeutisch wirksamen Dosis zwischen 100 µg und 5 g pro Tag bestimmt ist, und (b) eine Zusammensetzung, die Doxycyclin oder eines seiner pharmazeutisch annehmbaren Salze umfasst, die zur Verabreichung einer therapeutisch wirksamen Dosis von 40, 100 oder 200 mg pro Tag bestimmt ist, als Kombinationsprodukt zur gleichzeitigen, getrennten oder zeitlich gestaffelten Verwendung bei der Behandlung und/oder der Verzögerung des Auftretens von Rosacea-Symptomen.

2. Kit zur Verwendung nach Anspruch 1 bei der Behandlung und/oder der Verzögerung des Auftretens von Rosacea-Symptomen des Untertyps I, II und IV, vorzugsweise des Untertyps II.

3. Kit zur Verwendung nach einem der Ansprüche 1 oder 2 bei der Behandlung und/oder der Verzögerung des Auftretens von Symptomen einer Rosacea, die mit einer Resistenz von Demodex gegenüber Avermectinen, vorzugsweise Ivermectin, assoziiert ist/sie aufweist.

4. Kit zur Verwendung nach einem der Ansprüche 1 bis 3, **dadurch gekennzeichnet, dass** die Zusammensetzung, die Ivermectin enthält, zur topischen Verabreichung bestimmt ist.

5. Kit zur Verwendung nach einem der Ansprüche 1 bis 4, **dadurch gekennzeichnet, dass** die Zusammensetzung, die Doxycyclin oder eines seiner pharmazeutisch annehmbaren Salze enthält, zur oralen Verabreichung bestimmt ist.

6. Kit zur Verwendung nach einem der Ansprüche 1 bis 5, **dadurch gekennzeichnet, dass** die Zusammensetzung des Kits, die Doxycyclin oder eines seiner pharmazeutisch annehmbaren Salze enthält, zur Verabreichung einer therapeutisch wirksamen Dosis von 40 mg Doxycyclin pro Tag bestimmt ist.

## Claims

1. Kit comprising (a) a composition comprising ivermectin intended to be administered at a therapeutically effective dose of between 100 µg and 5 g per day, and (b) a composition comprising doxycycline or a pharmaceutically acceptable salt thereof, intended to be administered at a therapeutically effective dose of 40, 100, or 200 mg per day, as a combination product for its simultaneous, separate, or prolonged use in the treatment and/or delay of the onset of rosacea symptoms.

2. Kit for use according to claim 1, in the treatment and/or delay of the onset of symptoms of subtype 1, 2 and 4 rosacea, preferably subtype 2 rosacea,

3. Kit for use according to any of claims 1 or 2, in the treatment and/or delay of the onset of symptoms of rosacea associated with/exhibiting *Demodex* resistance to avermectins, preferably to ivermectin.

4. Kit for use according to any of claims 1 to 3, **characterized in that** the composition comprising ivermectin is intended for topical administration.

5. Kit for use according to any of claims 1 to 4, **characterized in that** the composition comprising doxycycline or a pharmaceutically acceptable salt thereof is intended for oral administration.

6. Kit for use according to any of claims 1 to 5, **characterized in that** the composition in the kit comprising doxycycline or a pharmaceutically acceptable salt thereof is intended to be administered at a therapeutically effective dose of 40 mg of doxycycline per day.
